Europäisches Patentamt

European Patent Office

Office européen des brevets

(11) Veröffentlichungsnummer: **0 510 358 B1**

## (12) EUROPÄISCHE PATENTSCHRIFT

(45) Veröffentlichungstag der Patentschrift: **31.08.94**

(21) Anmeldenummer: **92104741.1**

(22) Anmeldetag: **19.03.92**

Die Akte enthält technische Angaben, die nach
dem Eingang der Anmeldung eingereicht wurden
und die nicht in dieser Patentschrift enthalten sind.

(51) Int. Cl.⁵: **B01J 31/40**, C07C 45/50,
B01J 38/58

(54) **Verfahren zur Wiedergewinnung von Rhodium aus den Rückständen der Destillation von Produkten der Oxosynthese.**

(30) Priorität: **28.03.91 DE 4110212**

(43) Veröffentlichungstag der Anmeldung:
**28.10.92 Patentblatt 92/44**

(45) Bekanntmachung des Hinweises auf die
Patenterteilung:
**31.08.94 Patentblatt 94/35**

(84) Benannte Vertragsstaaten:
**AT BE DE ES FR GB GR IT NL SE**

(56) Entgegenhaltungen:
**EP-A- 0 255 673**
**EP-A- 0 424 736**
**EP-A- 0 475 036**
**US-A- 4 196 096**
**US-A- 4 578 368**

(73) Patentinhaber: **HOECHST AKTIENGESELL-
SCHAFT**

**D-65926 Frankfurt (DE)**

(72) Erfinder: **Lappe, Peter, Dr. Dipl.-Chem.
Eickenhof 34
W-4220 Dinslaken (DE)**
Erfinder: **Springer, Helmut, Dipl.-Chem.
Borbecker Strasse 19
W-4200 Oberhausen (DE)**

**Beschreibung**

Die vorliegende Erfindung betrifft ein verbessertes Verfahren zur Wiedergewinnung von Rhodium aus den Rückständen, die bei der Destillation von Produkten der Oxosynthese erhalten werden.

Die Herstellung von Aldehyden und Alkoholen durch Anlagerung von Kohlenmonoxid und Wasserstoff an olefinische Doppelbindungen (Hydroformylierung) ist bekannt. Die Reaktion wird durch Metalle der 8. Nebengruppe des Periodensystems oder deren Verbindungen katalysiert, die unter den Reaktionsbedingungen Carbonyle oder Hydridocarbonyle bilden. Während früher fast ausschließlich Kobalt und Kobaltverbindungen als Katalysatoren eingesetzt wurden, finden heute in zunehmendem Maße Rhodiumkatalysatoren Anwendung, obgleich Rhodium um ein vielfaches teurer als Kobalt ist. Rhodium gelangt dabei allein oder in Kombination mit Komplexbildnern, z.B. organischen Phosphinen, zum Einsatz. Während die Oxosynthese mit Rhodium als Katalysator Reaktionsdrücke von 25 bis 30 MPa erfordert, genügen bei Einsatz von Rhodium-Komplexverbindungen Drücke von 1 bis 5 MPa.

Für Rhodium-Katalysatoren ergeben sich in vielen Fällen deutliche Vorteile. Sie besitzen höhere Aktivität und Selektivität und ermöglichen überdies einen in vieler Hinsicht problemlosen Betrieb der Produktionsanlage, insbesondere was die Durchführung der Synthese und die Ausbringung der Produkte aus dem Reaktor anbetrifft. Schließlich kann das klassische Oxoverfahren auf Basis von Kobaltkatalysatoren unter Verwendung der vorhandenen Apparateteile in vielen Fällen mit nur geringen Investitionen auf Rhodiumkatalysatoren umgestellt werden.

Erhebliche Schwierigkeiten bereitet jedoch die verlustfreie oder zumindest annähernd verlustfreie Abtrennung und Wiedergewinnung des Rhodiums, unabhängig davon, ob es mit oder ohne zusätzlichem Komplexbildner als Katalysator eingesetzt wird. Nach Beendigung der Umsetzung findet sich das Rhodium als Carbonylverbindung, die gegebenenfalls noch weitere Liganden enthalten kann, gelöst im Hydroformylierungsprodukt.

Zur Aufarbeitung wird das Oxorohprodukt üblicherweise mehrstufig entspannt, indem man den Synthesedruck, der je nach Art des eingesetzten Rhodiumkatalysators etwa 1 bis 30 MPa beträgt, zunächst auf etwa 0,5 bis 2,5 MPa reduziert. Hierbei wird im Rohprodukt gelöstes Synthesegas frei. Anschließend kann man auf Normaldruck entspannen. Die Abtrennung des Rhodiums erfolgt entweder unmittelbar aus dem Rohprodukt oder aus dem Rückstand der Rohproduktdestillation. Der erste Weg wird dann beschritten, wenn in der vorausgegangenen Hydroformylierungsstufe Rhodium ohne zusätzlichen Komplexbildner als Katalyator eingesetzt worden war. Die zweite Variante wird angewandt, wenn der Rhodiumkatalysator außer Kohlenmonoxid noch weitere Liganden, z.B. Phosphine oder Phosphite in komplexer Bindung enthält. Sie kann auch dann genutzt werden, wenn die Hydroformylierung zwar mit Rhodium allein durchgeführt, dem Rohprodukt nach Entspannung aber ein Komplexbildner zur Stabilisierung des Rhodiums zugesetzt worden war. Grundsätzlich ist zu berücksichtigen, daß das Edelmetall im Rohprodukt in einer Konzentration von nur wenigen ppm vorliegt, seine Abtrennung daher sehr sorgfältiges Arbeiten erfordert. Zusätzliche Schwierigkeiten können dadurch entstehen, daß das Rhodium, insbesondere wenn es ohne Ligand eingesetzt wurde, bei der Entspannung teilweise in metallische Form übergeht oder mehrkernige Carbonyle bildet. Es kommt dann zur Ausbildung eines heterogenen Systems, das aus der flüssigen organischen und der festen, Rhodium oder Rhodiumverbindungen enthaltenden Phase besteht.

Die Rückgewinnung von Rhodium aus den Produkten der Oxosynthese, darunter auch den Rückständen von Oxorohprodukten, ist vielfach untersucht worden. Die Arbeiten führten zur Entwicklung zahlreicher Verfahren, von denen einige auch Anwendung im technischen Maßstab gefunden haben.

Gegenstand der US-PS 4 400 547 ist die Hydroformylierung von Olefinen mit 2 bis 20 Kohlenstoffatomen in Gegenwart von unmodifiziertem Rhodium als Katalysator. Nach Beendigung der Reaktion setzt man dem Oxorohprodukt eine komplexbildende Verbindung wie Triphenylphosphin zu und destilliert den Aldehyd ab. Anschließend wird der Destillationsrückstand mit Sauerstoff behandelt, um den Liganden aus der Komplexverbindung wieder abzuspalten und das Rhodium in aktiver Form zurückzugewinnen. Eine Trennung von Rhodium und Destillationsrückstand ist nach dieser Arbeitsweise nicht möglich.

Die Abtrennung von Edelmetallen wie Rhodium aus hochsiedenden Hydroformylierungsrückständen wird auch in der US-PS 3 547 964 beschrieben. Hierzu behandelt man die Rückstände in Gegenwart von Säuren wie Ameisensäure, Salpetersäure oder Schwefelsäure mit Wasserstoffperoxid. Wegen des hohen Preises von Wasserstoffperoxid und seiner problematischen Handhabung sind der technischen Anwendung des Verfahrens jedoch Grenzen gesetzt.

Nach der DE 24 48 005 C2 wird ein Rhodium enthaltender Destillationsrückstand zunächst mit Säuren und Peroxiden behandelt. Darauf zerstört man überschüssige Peroxide durch Erhitzen und setzt die das Katalysatormetall enthaltende wäßrige Lösung in Gegenwart eines wasserlöslichen organischen Lösungsmittels mit Halogenwasserstoffsäure oder Alkalihalogeniden sowie mit tertiären Phosphinen und Kohlenmonoxid oder Kohlenmonoxid abspaltenden Verbindungen um. Diese Arbeitsweise erfordert wiederum die Verwendung von Peroxiden mit den oben geschilderten Nachteilen und den Einsatz halogenbeständiger Werkstoffe.

In der US-PS 4 390 473 schließlich ist ein Verfahren zur Wiedergewinnung von Rhodium und Kobalt aus einer Lösung beschrieben, die als Katalysator in einem Niederdruck-Oxoverfahren eingesetzt worden war. Zur Abtrennung der komplex gebundenen Metalle gibt man zu der Lösung wäßrige Ameisensäure und leitet ein Sauerstoff enthaltendes Gas durch. Es entstehen zwei Phasen, eine organische und eine wäßrige, die die Metalle als Formiate gelöst enthält. Nach Trennung der Phasen können Kobalt und Rhodium aus der wäßrigen Lösung gewonnen werden. In der Praxis hat es sich jedoch als sehr störend erwiesen, daß die Ameisensäure reduzierend wirkt. Diese Eigenschaft führte dazu, daß Rhodium im Laufe des Verfahrens teilweise in metallischer Form abgeschieden wurde und nicht mehr wiedergewonnen werden konnte.

Es bestand daher die Aufgabe ein Verfahren zu entwickeln, das die geschilderten Nachteile vermeidet und auf recht einfachem Wege eine möglichst hohe Wiedergewinnung des Edelmetalls sicherstellt.

Erfindungsgemäß wird diese Aufgabe gelöst durch ein Verfahren zur Wiedergewinnung von Rhodium, das in Komplexverbindung mit einer organischen Phosphor-(III)-Verbindung in den Rückständen der Destillation von Produkten der Oxosynthese enthalten ist, durch Behandlung des Rückstandes mit Sauerstoff oder einem Sauerstoff enthaltenden Gas, dadurch gekennzeichnet, daß das den Rückstand enthaltende Einsatzmaterial 10 bis 1200 mol Aldehyd je g-Atom Rhodium enthält und man das Einsatzmaterial mit Sauerstoff oder einem Sauerstoff enthaltenden Gas bei 60 bis 120°C bei Normaldruck oder unter erhöhtem Druck in Gegenwart von 1,0 bis 15 mol einer Monocarbonsäure mit 2 bis 5 Kohlenstoffatomen je g-Atom Rhodium und in Gegenwart eines Alkalisalzes einer Monocarbonsäure mit 2 bis 5 Kohlenstoffatomen behandelt und anschließend zur Abtrennung des Rhodiums als wasserlösliche Verbindung mit Wasser extrahiert und darauf die wäßrige und die organische Phase voneinander trennt.

Die erfindungsgemäße Arbeitsweise erfordert weder einen großen apparativen Aufwand noch den Gebrauch wertvoller Chemikalien. Trotz eines vergleichsweise geringen Einsatzes von Hilfsstoffen führt sie überraschenderweise zur Wiedergewinnung von weit mehr als 90 % des eingesetzten Rhodiums. Das zurückgewonnene Rhodium läßt sich ohne besondere zusätzliche Maßnahmen unmittelbar als Katalysator oder Katalysatorbestandteil in einer Hydroformylierungsreaktion verwenden, wobei zugleich sichergestellt ist, daß eine Bildung unerwünschter Nebenprodukte während der Hydroformylierung weitestgehend vermieden wird.

Das neue Verfahren geht von den Rückständen der Hydroformylierung olefinisch ungesättigter Verbindungen aus, wie sie nach destillativer Abtrennung der Hydroformylierungsprodukte als Destillationsrückstand anfallen. Sie enthalten neben unterschiedlichen Mengen Aldehyden im wesentlichen höhermolekulare Verbindungen, die unter anderem aus Aldehyden durch Aldolreaktion entstanden sind und in einer Folgereaktion entweder unter Abspaltung von Wasser ungesättigte Verbindungen bilden können oder in Umkehrung der Aldolreaktion zur Entstehung von Aldehyden führen können.

Für das Gelingen des erfindungsgemäßen Verfahrens ist es von Bedeutung, den Aldehydgehalt im Rückstand oder in dem den Rückstand enthaltenden Einsatzmaterial zu begrenzen. Dies erreicht man üblicherweise durch eine zusätzliche destillative Abtrennung aldehydischer Verbindungen aus dem Rückstand. Der derart behandelte Rückstand kann direkt in das erfindungsgemäße Verfahren eingesetzt werden, vorausgesetzt, er eignet sich aufgrund seiner Zusammensetzung, insbesondere aufgrund seines Rhodiumgehaltes hierfür. In den meisten Fällen ist der Rückstand durch Zusatz eines Lösungsmittels zu verdünnen. Die dabei anfallende Lösung dient als das den Rückstand enthaltende Einsatzmaterial.

Der Rückstand oder das den Rückstand enthaltende Einsatzmaterial soll nicht mehr als 1200, insbesondere 10 bis 1200, bevorzugt 30 bis 1100 mol Aldehyd je g-Atom Rhodium enthalten.

Die Begrenzung des Aldehydgehaltes ist in erheblichem Umfange auch von der Art des im Rückstand oder im Einsatzmaterial enthaltenen Aldehyds abhängig. Kurzkettige Aldehyde mit drei bis fünf Kohlenstoffatomen erfordern eine relativ weitgehende Abtrennung, langkettige Aldehyde können in größerer Menge vorhanden sein. Enthält der Rückstand oder das den Rückstand enthaltende Einsatzmaterial Propionaldehyd, so soll ein Verhältnis von 10 bis 500, insbesondere 20 bis 400, bevorzugt 30 bis 300 mol Propionaldehyd je g-Atom Rhodium eingehalten werden. Weist der Rückstand oder das den Rückstand enthaltende Einsatzmaterial α-Formylpropionsäuremethylester als Aldehyd auf, so ist ein Verhältnis von 100 bis 800, insbesondere 200 bis 700, bevorzugt 300 bis 600 mol Aldehyd je g-Atom Rhodium zulässig. Im Falle eines Aldehyds mit 9 Kohlenstoffatomen (hergestellt durch Hydroformylierung von Diisobutylen) kann das Verhältnis 600 bis 1200, insbesondere 700 bis 1150, bevorzugt 800 bis 1100 mol Aldehyd je g-Atom Rhodium betragen.

Höhere Aldehydanteile führen im allgemeinen zu einer deutlichen Verschlechterung der Wiedergewinnung von Rhodium und erfordern zudem stark ansteigende Mengen der zuzusetzenden Carbonsäure und des Carbonsäuresalzes. Dies wirkt sich allerdings auf die Wiedereinsetzbarkeit des zurückgewonnenen Rhodiums als Hydroformylierungskatalysator oder Hydroformylierungskatalysatorbestandteil ungünstig aus.

Die Art der Verbindungen, die hydroformyliert wurden, ist für die beanspruchte Arbeitsweise ohne Bedeutung. Dementsprechend können sowohl Rückstände, die aus der Reaktion von Olefinen mit Kohlenmonoxid und Wasserstoff resultieren, als auch höhermolekulare Produkte, die bei der Umsetzung olefinisch ungesättigter Verbindungen entstehen, die außer der Doppelbindung noch funktionelle Gruppen im Molekül enthalten, eingesetzt werden. Im Vordergrund des neuen Verfahrens steht aber die Wiedergewinnung von Rhodium aus den Rückständen der Hydroformylierung von Olefinen mit 2 bis 12 Kohlenstoffatomen, entsprechend der wirtschaftlichen Bedeutung der aus ihnen hergestellten Aldehyde. Neben den gesättigten und ungesättigten Kondensationsprodukten können die Rückstände auch noch Verbindungen enthalten, die mit den Rhodiumionen unter Komplexbildung reagieren und gegenüber dem Rhodium zumeist im Überschuß vorhanden sind. Zu diesen Verbindungen gehören organische Phosphor(III)-verbindungen, insbesondere Phosphine und Phosphite, vorzugsweise die Arylverbindungen wie Triphenylphosphin und Triphenylphosphit. Ihre Aufgabe ist es, durch Bildung stabiler Komplexverbindungen während der Umsetzung die Selektivität der Reaktion zu verbessern und nach der Umsetzung die Abscheidung metallischen Rhodiums zu verhindern. Im Reaktionsgemisch beträgt das Verhältnis von Ligand und Rhodium 2 bis 150, insbesondere 5 bis 50 mol/g-Atom. Wegen ihrer geringen Flüchtigkeit liegen beide Komponenten auch im Destillationsrückstand etwa im selben Verhältnis vor, wobei die Rhodiumkonzentration zwischen 30 und 1000 Masse-ppm, insbesondere 100 bis 500 Masse-ppm beträgt.

Erfindungsgemäß wird der Destillationsrückstand oder das den Destillationsrückstand enthaltende Einsatzmaterial mit Sauerstoff behandelt. Das Oxidationsmittel wird in reiner Form oder als Sauerstoff enthaltendes Gasgemisch, insbesondere Luft eingesetzt. Die Sauerstoffmenge kann in weiten Grenzen variiert werden. Sie richtet sich vorzugsweise nach der Menge des Einsatzmaterials. Es empfiehlt sich je kg Einsatzmaterial 1 bis 10, insbesondere 2 bis 6 mol Sauerstoff anzuwenden.

Entsprechend der Erfindung erfolgt die Behandlung des Destillationsrückstandes oder des den Rückstand enthaltenden Einsatzmaterials mit Sauerstoff in Gegenwart einer gesättigten, geradkettigen oder verzweigten Monocarbonsäure mit 2 bis 5 Kohlenstoffatomen.

Beispiele für geeignete Säuren sind Essigsäure, Propionsäure, n-Buttersäure, i-Buttersäure und n-Valeriansäure. Besonders bewährt haben sich Essigsäure und Propionsäure. Sie werden in handelsüblicher Form und in einer solchen Menge eingesetzt, daß je g-At Rhodium etwa 1,0 bis 15, insbesondere 1,2 bis 10, bevorzugt 1,5 bis 6 mol vorhanden sind. Die Säure wird dem Rückstand oder dem den Rückstand enthaltenden Einsatzmaterial vor der Umsetzung mit Sauerstoff zugesetzt, unabhängig davon, daß sich aufgrund der begrenzten Menge Aldehyd, die im Rückstand noch vorhanden ist, im Laufe der Reaktion aus dem Rückstand selbst Säure bilden kann. Die genaue Wirkungsweise der Säure ist nicht bekannt. Verschiedene Beobachtungen sprechen dafür, daß sie eine Startfunktion ausübt, d.h. das Einsetzen der Reaktion maßgebend beeinflußt.

Ein weiteres, ebenfalls sehr wichtiges Merkmal des erfindungsgemäßen Verfahrens ist die Anwesenheit eines Alkalicarboxylats im Rückstand oder in dem den Rückstand enthaltenden Einsatzmaterial, während Sauerstoff in das Gemisch der hochsiedenden Verbindungen eingeleitet wird. Auch die Art seines Eingreifens in das Reaktionsgeschehen ist nicht eindeutig zu erklären. Es hat sich jedoch gezeigt, daß der Carboxylatzusatz eine deutliche Erhöhung der zurückgewonnenen Rhodiummenge, d.h. eine weitere Verminderung des in der organischen Phase gelöst bleibenden Rhodiums zur Folge hat. Als Alkalicarboxylate werden im Rahmen des neuen Verfahrens Salze gesättigter, geradkettiger oder verzweigter Monocarbonsäuren mit 2 bis 5 Kohlenstoffatomen eingesetzt. Besonders bewährt haben sich die Natrium- und Kaliumsalze der Essigsäure, der Propionsäure, der n- und iso-Buttersäure und der n-Valeriansäure. Sie werden in einer Menge von 10 bis 100, insbesondere 12 bis 50, bevorzugt 12 bis 30 mol je g-At Rhodium angewandt. Geeignet sind die handelsüblichen Salze, die jedoch erst im Verlauf der Oxidation allmählich in Lösung gehen. Vorteilhafter ist es daher, zum Rückstand oder zu dem den Rückstand enthaltenden Einsatzmaterial freie Säure und die äquivalente Menge Alkalihydroxid zu geben, die sofort homogen gelöst und damit voll wirksam werden.

Die Umsetzung des Rückstandes mit Sauerstoff erfolgt bei 60 bis 120, insbesondere 70 bis 105, bevorzugt 80 bis 100 °C. Sie kann bei Normaldruck oder unter erhöhtem Druck vorgenommen werden, Drücke zwischen 0,2 und 1,0 MPa haben sich besonders bewährt.

Nach einer bevorzugten Ausführungsform des erfindungsgemäßen Verfahrens enthält das mit Sauerstoff zu behandelnde Einsatzmaterial Rhodium in einer Konzentration von etwa 200 Masse-ppm und weniger, insbesondere 20 bis 200, bevorzugt 30 bis 150 Masse-ppm. Es wurde nämlich gefunden, daß die Rhodiumrestmengen im erfindungsgemäß behandelten Rückstand dann besonders niedrig sind, wenn die Rhodiumkonzentration in der Ursprungslösung (Einsatzmaterial) innerhalb der genannten Bereiche liegt. Es empfiehlt sich daher Rückstände, in denen die Rhodiumkonzentration mehr als etwa 300 Masse-ppm beträgt, entsprechend zu verdünnen und diese Lösungen als Rückstand enthaltendes Einsatzmaterial zu verwenden. Als Verdünnungsmittel geeignet sind insbesondere höher siedende aliphatische oder aromatische Kohlenwasserstoffe, beispielsweise Toluol und Xylol, oder Kohlenwasserstoffgemische oder auch vom Rhodium-Katalysator befreite Destillationsrückstände.

Die Reaktionszeit ist abhängig von der Rhodium- und der Ligandenkonzentration im Destillationsrückstand. Sie wird weiterhin durch die eingesetzte Sauerstoffmenge, durch Reaktionstemperatur und -druck bestimmt. Hohe Konzentrationen der gelösten Substanzen erfordern längere Behandlungszeiten als niedrige Konzentrationen. Ein großes Sauerstoffangebot und erhöhter Druck vermindern die Reaktionszeit ebenso wie eine intensive Durchmischung des Rückstandes mit Sauerstoff. Temperaturen im unteren und im oberen Bereich des beanspruchten Intervalls sind etwas weniger effektiv als im mittleren Temperaturbereich.

Die Umsetzung des Destillationsrückstandes kann in konventionellen Apparaturen kontinuierlich oder diskontinuierlich durchgeführt werden. Der Sauerstoff oder das Sauerstoff enthaltende Gas wird über Verteilungsvorrichtungen in den Reaktor geleitet, die gleichmäßige Vermischung von flüssiger und gasförmiger Phase gegebenenfalls durch Rühren unterstützt.

Nach Abschluß der Behandlung mit Sauerstoff wird die organische Phase mit Wasser extrahiert. Man arbeitet bei Raumtemperatur oder erhöhter Temperatur, beispielsweise 50 bis 90°C, in einer, zweckmäßiger in mehreren Stufen. Die eingesetzte Wassermenge richtet sich nach dem Verteilungsgleichgewicht der zu extrahierenden Substanz zwischen organischer und wäßriger Phase und der angestrebten Rhodiumkonzentration in der wäßrigen Phase. Die wäßrige Lösung der Rhodiumverbindung kann auch durch Kreisführung wiederholt zur Extraktion verwendet werden, um dadurch eine Anreicherung des Metalles in der Lösung zu erzielen. Die wäßrige Lösung kann ohne zusätzliche Reinigungsschritte unmittelbar zur Katalysatorherstellung verwendet werden.

Die folgenden Beispiele erläutern die Erfindung, ohne sie auf diese Ausführungsformen zu beschränken.

In der nachfolgenden Tabelle sind die Einsatzstoffe (Rückstände) durch ihre wesentlichen Kennzahlen charakterisiert.

**Tabelle**

| | Rückstände aus der Propionaldehyd-Synthese (Vergleich) | | | | | | | | | | α-FPSME* Vergleich | | i-C$_9$-Aldehyd** Vergleich | | |
|---|---|---|---|---|---|---|---|---|---|---|---|---|---|---|---|
| | A | B | C | D | E | F | G | H | I | J | K | L | M | N | O |
| Rh-Gehalt (Masse-ppm) | 516 | 504 | 992 | 374 | 537 | 338 | 110 | 155 | 177 | 209 | 430 | 334 | 251 | 191 | 154 |
| P(III)-Gehalt (mmol/kg) | 64 | 85 | 75 | 51 | 61 | 46 | 17 | 22 | 28 | 24 | 13 | 10 | 61 | 48 | 33 |
| P gesamt (mmol/kg) | 97 | 91 | 131 | 70 | 82 | 48 | 18 | 29 | 33 | 28 | 33 | 24 | 65 | 51 | 37 |
| Neutralisationszahl (mg KOH/g) | 18 | 19 | 17 | 14 | 19 | 15 | – | 9 | 10 | 10 | 66 | 46 | 10 | 10 | 8 |
| Wasser (%) | 0,21 | 0,18 | 0,26 | 0,17 | 0,17 | 0,14 | 0,09 | 0,10 | 0,10 | 0,10 | 0,04 | 0,08 | 0,27 | 0,19 | 0,16 |
| aldehydische Komponenten (mmol/kg) | 871 | 1022 | 457 | 1063 | 846 | 1160 | 2137 | 3008 | 2566 | 2669 | 2155 | 5510 | 2391 | 3501 | 4182 |
| Verhältnis Aldehyd:Rh (mol/g-Atom) | 174 | 209 | 47 | 292 | 162 | 353 | 1999 | 1997 | 1492 | 1314 | 516 | 1697 | 980 | 1886 | 2792 |

\* Rückstand aus der α-Formylpropionsäuremethylester-Synthese (Hydroformylierung von Acrylsäuremethylester)

\*\* Rückstand aus der i-Nonylaldehyd-Synthese (Hydroformylierung von Diisobutylen)

Experimenteller Teil

Die Rückstände A bis J entstammen der Herstellung von Propionaldehyd, die Rückstände K bis M der Herstellung von α-Formylpropionsäuremethylester (α-FPSME) und die Rückstände M bis O der Herstellung von i-Nonylaldehyd.

Unter Einsatzmaterial versteht man das Gemisch aus dem jeweils eingesetzten Destillationsrückstand und Lösungsmittel.

Beispiel 1

Rückstand ex Propionaldehyd-Synthese

| Einsatzstoffe: | |
| --- | --- |
| Destillationsrückstand A: | 81,4 g ($\triangleq$ 42 mg Rh) |
| Xylol: | 518,6 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Die obengenannten Einsatzstoffe werden in einem mit einer Mantelbeheizung versehenen Glasautoklaven (Volumen: 1 l) vorgelegt und unter Rühren innerhalb von 15 Minuten auf 95°C erwärmt. Anschließend leitet man durch ein Tauchrohr über einen Zeitraum von 6 Stunden unter einem Druck von 0,35 MPa je Stunde 50 l Luft (entsprechend 4,0 mol $O_2$/kg Einsatzmaterial) ein. Die Umsetzung erfolgt bei konstantem Innendruck von 0,35 MPa und konstanter Temperatur von 100°C. Über ein Nadelventil im Deckel des Autoklaven wird das Abgas abgeführt und in einen mit Kühler versehenen Kolben geleitet.

Nach Abschluß der Reaktion wird der Inhalt des Autoklaven innerhalb von etwa 15 Minuten auf 80°C abgekühlt und die Zufuhr von Luft unterbrochen. Man entspannt, versetzt das Reaktionsgemisch mit 600 g Wasser und rührt weitere 15 Minuten bei 70 bis 80°C, entnimmt das Gemisch dem Autoklaven, trennt die wäßrige Phase von der organischen Phase und extrahiert die organische Phase noch einmal mit 600 g Wasser.

Die organische Phase enthält noch 0,72 mg Rh, d.h. 1,7 Masse-% des ursprünglich eingesetzten Rhodiums.

Beispiel 2

Rückstand ex Propionaldehyd-Synthese

| Einsatzstoffe: | |
| --- | --- |
| Destillationsrückstand B: | 83,3 g ($\triangleq$ 42 mg Rh) |
| Xylol: | 516,7 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben, jedoch mit 40 l Luft je Stunde (entsprechend 3,2 mol $O_2$/kg Einsatzmaterial)

Die organische Phase enthält noch 1,32 mg Rh, d.h. 3,1 Masse-% des ursprünglich eingesetzten Rhodiums.

Beispiel 3

Rückstand ex Propionaldehyd-Synthese

| Einsatzstoffe: | |
|---|---|
| Destillationsrückstand C: | 42,3 g ($\triangleq$ 42 mg Rh) |
| Xylol: | 557,7 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben, jedoch mit 38 l Luft je Stunde (entsprechend 3,0 mol $O_2$/kg Einsatzmaterial).

Die organische Phase enthält noch 0,28 mg Rh, d.h. 0,7 Masse-% des ursprünglich eingesetzten Rhodiums.

Beispiel 4

Rückstand ex Propionaldehyd-Synthese

| Einsatzstoffe: | |
|---|---|
| Destillationsrückstand D: | 112,3 g ($\triangleq$ 42 mg Rh) |
| Xylol: | 487,7 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben, jedoch mit 45 l Luft je Stunde (entsprechend 3,6 mol $O_2$/kg Einsatzmaterial).

Die organische Phase enthält noch 0,89 mg Rh, d.h. 2,1 Masse-% des ursprünglich eingesetzten Rhodiums.

Beispiel 5

Rückstand ex Propionaldehyd-Synthese

| Einsatzstoffe: | |
|---|---|
| Destillationsrückstand E: | 78,2 g ($\triangleq$ 42 mg Rh) |
| Xylol: | 521,8 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben, jedoch mit 30 l Luft je Stunde (entsprechend 2,4 mol $O_2$/kg Einsatzmaterial).

Die organische Phase enthält noch 0,26 mg Rh, d.h. 0,6 Masse-% des ursprünglich eingesetzten Rhodiums.

Beispiel 6

Rückstand ex Propionaldehyd-Synthese

| Einsatzstoffe: | |
|---|---|
| Destillationsrückstand E: | 156,4 g ($\triangleq$ 84 mg Rh) |
| Xylol: | 443,6 g |
| NaOH (30 %ige Lösung): | 2,72 g (20,4 mmol) |
| Propionsäure (99,5 %ig): | 1,72 g (23,2 mmol) |
| Verhältnis Rh(g-Atom)/Carbonsäure (mol)/Carbonsäuresalz (mol): | 1 : 3 : 25 |

Man arbeitet wie in Beispiel 1 angegeben.

Die organische Phase enthält noch 2,09 mg Rh, d.h. 2,5 Masse-% des ursprünglich eingesetzten Rhodiums.

Beispiel 7

Rückstand ex Propionaldehyd-Synthese

| Einsatzstoffe: | |
|---|---|
| Destillationsrückstand E: | 234,6 g ($\triangleq$ 126 mg Rh) |
| Xylol: | 365,4 g |
| NaOH (30 %ige Lösung): | 4,08 g (30,6 mmol) |
| Propionsäure (99,5 %ig): | 2,58 g (34,7 mmol) |
| Verhältnis Rh(g-Atom)/Carbonsäure (mol)/Carbonsäuresalz (mol): | 1 : 3 : 25 |

Man arbeitet wie in Beispiel 1 angegeben.

Die organische Phase enthält noch 4,34 mg Rh, d.h. 3,4 Masse-% des ursprünglich eingesetzten Rhodiums.

Beispiel 8

Rückstand ex Propionaldehyd-Synthese

| Einsatzstoffe: | |
|---|---|
| Destillationsrückstand F: | 124,3 g ($\triangleq$ 42 mg Rh) |
| Toluol: | 475,7 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben, jedoch bei 95 °C.

Die organische Phase enthält noch 1,29 mg Rh, d.h. 3,1 Masse-% des ursprünglich eingesetzten Rhodiums.

Beispiel 9 (Vergleich)

Rückstand ex Propionaldehyd-Synthese

| Einsatzstoffe: | |
|---|---|
| Destillationsrückstand G: | 381,8 g (≙ 42 mg Rh) |
| Xylol: | 218,2 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben.

Die organische Phase enthält noch 4,49 mg Rh, d.h. 10,7 Masse-% des ursprünglich eingesetzten Rhodiums. Das Verhältnis Aldehyd (mol) : Rh (g-Atom) liegt bei 1999 (vgl. Tabelle).

Beispiel 10 (Vergleich)

Rückstand ex Propionaldehyd-Synthese

| Einsatzstoffe: | |
|---|---|
| Destillationsrückstand H: | 271 g (≙ 42 mg Rh) |
| Xylol: | 329 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben.

Die organische Phase enthält noch 2,94 mg Rh, d.h. 7,0 Masse-% des ursprünglich eingesetzten Rhodiums. Das Verhältnis Aldehyd : Rh liegt bei 1997 (vgl. Tabelle).

Beispiel 11 (Vergleich)

Rückstand ex Propionaldehyd-Synthese

| Einsatzstoffe: | |
|---|---|
| Destillationsrückstand I: | 237,3 g (≙ 42 mg Rh) |
| Toluol: | 362,7 g |
| NaOH (30 %ige Lösung): | 8,16 g (61,2 mmol) |
| Propionsäure (99,5 %ig): | 5,14 g (69,2 mmol) |
| Verhältnis Rh(g-Atom)/Carbonsäure (mol)/Carbonsäuresalz (mol): | 1 : 20 : 150 |

Man arbeitet wie in Beispiel 1 angegeben, jedoch mit 45 l Luft je Stunde (entsprechend 3,6 mol $O_2$/kg Einsatzmaterial)

Die organische Phase enthält noch 3,97 mg Rh, d.h. 9,5 Masse-% des ursprünglich eingesetzten Rhodiums. Das Verhältnis Aldehyd : Rh liegt bei 1492 (vgl. Tabelle).

Beispiel 12 (Vergleich)

Rückstand ex Propionaldehyd-Synthese

| Einsatzstoffe: | |
|---|---|
| Destillationsrückstand J: | 201 g ($\hat{=}$ 42 mg Rh) |
| Toluol: | 399 g |
| NaOH (30 %ige Lösung): | 8,16 g (61,2 mmol) |
| Propionsäure (99,5 %ig): | 5,14 g (69,1 mmol) |
| Verhältnis Rh(g-Atom)/Carbonsäure (mol)/Carbonsäuresalz (mol): | 1 : 20 : 150 |

Man arbeitet wie in Beispiel 1 angegeben, jedoch mit 40 l Luft je Stunde (entsprechend 3,2 mol $O_2$/kg Einsatzmaterial.

Die organische Phase enthält noch 2,89 mg Rh, d.h. 6,9 Masse-% des ursprünglich eingesetzten Rhodiums. Das Verhältnis Aldehyd : Rh liegt bei 1314 (vgl. Tabelle).

Beispiel 13

Rückstand ex α-Formylpropionsäuremethylester-Synthese

| Einsatzstoffe: | |
|---|---|
| Destillationsrückstand K: | 97,7 g ($\hat{=}$ 42 mg Rh) |
| Xylol: | 502,3 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben, jedoch bei 80°C und mit 40 l Luft je Stunde (entsprechend 3,2 mol $O_2$/kg Einsatzmaterial).

Die organische Phase enthält noch 1,39 mg Rh, d.h. 3,3 Masse-% des ursprünglich eingesetzten Rhodiums.

Beispiel 14

Rückstand ex α-Formylpropionsäuremethylester-Synthese

| Einsatzstoffe: | |
|---|---|
| Destillationsrückstand K: | 97,7 g ($\hat{=}$ 42 mg Rh) |
| Xylol: | 502,3 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben, jedoch bei 95°C und mit 40 l Luft je Stunde (entsprechend 3,2 mol $O_2$/kg Einsatzmaterial).

Die organische Phase enthält noch 0,31 mg Rh, d.h. 0,7 Masse-% des ursprünglich eingesetzten Rhodiums.

Beispiel 15 (Vergleich)

Rückstand ex α-Formylpropionsäuremethylester-Synthese

| Einsatzstoffe: | |
|---|---|
| Destillationsrückstand L: | 125,7 g (≙ 42 mg Rh) |
| Xylol: | 474,3 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 3 : 25 |

Man arbeitet wie in Beispiel 1 angegeben, jedoch bei 80°C und mit 40 l Luft je Stunde (entsprechend 3,2 mol $O_2$/kg Einsatzmaterial).

Die organische Phase enthält noch 3,79 mg Rh, d.h. 9,0 Masse-% des ursprünglich eingesetzten Rhodiums. Das Verhältnis Aldehyd : Rh liegt bei 1697 (vgl. Tabelle).

Beispiel 16

Rückstand ex i-Nonylaldehyd-Synthese

| Einsatzstoffe: | |
|---|---|
| Destillationsrückstand M: | 159,1 g (≙ 40 mg Rh) |
| Toluol: | 440,9 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 4 : 26 |

Man arbeitet wie in Beispiel 1 angegeben, jedoch bei 90°C und mit 40 l Luft je Stunde (entsprechend 3,2 mol $O_2$/kg Einsatzmaterial).

Die organische Phase enthält noch 1,26 mg Rh, d.h. 3,2 Masse-% des ursprünglich eingesetzten Rhodiums.

Beispiel 17

Rückstand ex i-Nonylaldehyd-Synthese

| Einsatzstoffe: | |
|---|---|
| Destillationsrückstand M: | 159,1 g (≙ 40 mg Rh) |
| Toluol: | 440,9 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 4 : 26 |

Man arbeitet wie in Beispiel 1 angegeben, jedoch mit 40 l Luft je Stunde (entsprechend 3,2 mol $O_2$/kg Einsatzmaterial).

Die organische Phase enthält noch 0,8 mg Rh, d.h. 2,0 Masse-% des ursprünglich eingesetzten Rhodiums.

Beispiel 18

Rückstand ex i-Nonylaldehyd-Synthese

| Einsatzstoffe: | |
|---|---|
| Destillationsrückstand M: | 159,1 g ($\triangleq$ 40 mg Rh) |
| Toluol: | 440,9 g |
| NaOH (30 %ige Lösung): | 0,82 g (6,15 mmol) |
| Propionsäure (99,5 %ig): | 0,52 g (7,0 mmol) |
| Verhältnis Rh(g-Atom)/Carbonsäure (mol)/Carbonsäuresalz (mol): | 1 : 2 : 16 |

Man arbeitet wie in Beispiel 1 angegeben, jedoch mit 40 l Luft je Stunde (entsprechend 3,2 mol $O_2$/kg Einsatzmaterial).

Die organische Phase enthält noch 1,39 mg Rh, d.h. 3,5 Masse-% des ursprünglich eingesetzten Rhodiums.

Beispiel 19 (Vergleich)

Rückstand ex i-Nonylaldehyd-Synthese

| Einsatzstoffe: | |
|---|---|
| Destillationsrückstand N: | 209,1 g ($\triangleq$ 40 mg Rh) |
| Toluol: | 390,9 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 4 : 26 |

Man arbeitet wie in Beispiel 1 angegeben, jedoch mit 40 l Luft je Stunde (entsprechend 3,2 mol $O_2$/kg Einsatzmaterial).

Die organische Phase enthält noch 2,89 mg Rh, d.h. 7,2 Masse-% des ursprünglich eingesetzten Rhodiums. Das Verhältnis Aldehyd : Rh liegt bei 1886 (vgl. Tabelle).

Beispiel 20 (Vergleich)

Rückstand ex i-Nonylaldehyd-Synthese

| Einsatzstoffe: | |
|---|---|
| Destillationsrückstand O: | 259,1 g ($\triangleq$ 40 mg Rh) |
| Toluol: | 340,9 g |
| NaOH (30 %ige Lösung): | 1,36 g (10,2 mmol) |
| Propionsäure (99,5 %ig): | 0,86 g (11,6 mmol) |
| Verhältnis Rh(g-Atom)/Carbonsäure (mol)/Carbonsäuresalz (mol): | 1 : 4 : 26 |

Man arbeitet wie in Beispiel 1 angegeben, jedoch mit 40 l Luft je Stunde (entsprechend 3,2 mol $O_2$/kg Einsatzmaterial).

Die organische Phase enthält noch 5,23 mg Rh, d.h. 13,1 Masse-% des ursprünglich eingesetzten Rhodiums. Das Verhältnis Aldehyd : Rh liegt bei 2792 (vgl. Tabelle).

**Patentansprüche**

1. Verfahren zur Wiedergewinnung von Rhodium, das in Komplexverbindung mit einer organischen Phosphor-(III)-Verbindung in den Rückständen der Destillation von Produkten der Oxosynthese enthalten ist, durch Behandlung des Rückstandes mit Sauerstoff oder einem Sauerstoff enthaltenden Gas, dadurch gekennzeichnet, daß das den Rückstand enthaltende Einsatzmaterial 10 bis 1200 mol Aldehyd je g-Atom Rhodium enthält und man das Einsatzmaterial mit Sauerstoff oder einem Sauerstoff enthaltenden Gas bei 60 bis 120°C bei Normaldruck oder unter erhöhtem Druck in Gegenwart von 1,0 bis 15 mol einer Monocarbonsäure mit 2 bis 5 Kohlenstoffatomen je g-Atom Rhodium und in Gegenwart eines Alkalisalzes einer Monocarbonsäure mit 2 bis 5 Kohlenstoffatomen behandelt und anschließend zur Abtrennung des Rhodiums als wasserlösliche Verbindung mit Wasser extrahiert und darauf die wäßrige und die organische Phase voneinander trennt.

2. Verfahren nach Anspruch 1, dadurch gekennzeichnet, daß als Sauerstoff enthaltendes Gas Luft verwendet wird.

3. Verfahren nach Anspruch 1 oder 2, dadurch gekennzeichnet, daß je kg Einsatzmaterial 1 bis 10, insbesondere 2 bis 6 mol Sauerstoff angewendet werden.

4. Verfahren nach einem oder mehreren der Ansprüche 1 bis 3, dadurch gekennzeichnet, daß dem Einsatzmaterial Essigsäure, Propionsäure, n-Buttersäure, i-Buttersäure oder n-Valeriansäure zugesetzt wird.

5. Verfahren nach einem oder mehreren der Ansprüche 1 bis 4, dadurch gekennzeichnet, daß dem Einsatzmaterial je g-Atom Rhodium 1,2 bis 10, insbesondere 1,5 bis 6 mol Monocarbonsäure zugesetzt wird.

6. Verfahren nach einem oder mehreren der Ansprüche 1 bis 5, dadurch gekennzeichnet, daß dem Einsatzmaterial ein Natrium- oder Kaliumsalz der Essigsäure, Propionsäure, n-Buttersäure, i-Buttersäure oder n-Valeriansäure zugesetzt wird.

7. Verfahren nach einem oder mehreren der Ansprüche 1 bis 6, dadurch gekennzeichnet, daß dem Einsatzmaterial je g-Atom Rhodium 10 bis 100, insbesondere 12 bis 50, bevorzugt 12 bis 30 mol Alkalisalz der Monocarbonsäure mit 2 bis 5 Kohlenstoffatomen zugesetzt wird.

8. Verfahren nach einem oder mehreren der Ansprüche 1 bis 7, dadurch gekennzeichnet, daß die Behandlung des Einsatzmaterials mit Sauerstoff oder einem Sauerstoff enthaltenden Gas bei 70 bis 105, bevorzugt 80 bis 100°C erfolgt.

9. Verfahren nach einem oder mehreren der Ansprüche 1 bis 8, dadurch gekennzeichnet, daß die Behandlung des Einsatzmaterials mit Sauerstoff oder einem Sauerstoff enthaltenden Gas bei Drücken von 0,2 bis 1,0 MPa erfolgt.

10. Verfahren nach einem oder mehreren der Ansprüche 1 bis 9, dadurch gekennzeichnet, daß die Rhodiumkonzentration des Einsatzmaterials etwa 300 Masse-ppm und weniger, insbesondere 20 bis 200, bevorzugt 30 bis 150 Masse-ppm beträgt.

**Claims**

1. A process for the recovery of rhodium, which is contained in complex linkage with an organic phosphorus(III) compound in the residues of the distillation of the products of the oxo synthesis, by treatment of the residue with oxygen or an oxygen-containing gas, wherein the starting material containing the residue contains 10 to 1200 mol of aldehyde per g atom of rhodium, and the starting material is treated with oxygen or an oxygencontaining gas at 60 to 120°C under atmospheric pressure or under elevated pressure in the presence of 1.0 to 15 mol of a monocarboxylic acid with 2 to 5 carbon atoms per g atom of rhodium and in the presence of an alkali metal salt of a monocarboxylic acid with 2 to 5 carbon atoms, and subsequently extracted with water to remove the rhodium as water-soluble compound, and then the aqueous is separated from the organic phase.

2. The process as claimed in claim 1, wherein air is used as oxygen-containing gas.

3. The process as claimed in claim 1 or 2, wherein 1 to 10, in particular 2 to 6, mol of oxygen are used per kg of starting material.

4. The process as claimed in one or more of claims 1 to 3, wherein acetic acid, propionic acid, n-butyric acid, i-butyric acid or n-valeric acid is added to the starting material.

5. The process as claimed in one or more of claims 1 to 4, wherein 1.2 to 10, in particular 1.5 to 6, mol of monocarboxylic acid are added to the starting material per g atom of rhodium.

6. The process as claimed in one or more of claims 1 to 5, wherein a sodium or potassium salt of acetic acid, propionic acid, n-butyric acid, i-butyric acid or n-valeric acid is added to the starting material.

7. The process as claimed in one or more of claims 1 to 6, wherein 10 to 100, in particular 12 to 50, preferably 12 to 30, mol of alkali metal salt of the monocarboxylic acid with 2 to 5 carbon atoms are added to the starting material per g atom of rhodium.

8. The process as claimed in one or more of claims 1 to 7, wherein the treatment of the starting material with oxygen or an oxygen-containing gas is carried out at 70 to 105, preferably 80 to 100°C.

9. The process as claimed in one or more of claims 1 to 8, wherein the treatment of the starting material with oxygen or an oxygen-containing gas is carried out under pressures of from 0.2 to 1.0 MPa.

10. The process as claimed in one or more of claims 1 to 9, wherein the rhodium concentration in the starting material is about 300 ppm by weight and less, in particular 20 to 200, preferably 30 to 150 ppm by weight.

**Revendications**

1. Procédé de récupération de rhodium qui se trouve sous forme d'un composé organique de phosphore (III) dans les résidus de distillation de produits de synthèse oxo, par traitement du résidu par de l'oxygène ou par un gaz contenant de l'oxygène, caractérisé en ce que le matériau mis en oeuvre contenant le résidu contient 10 à 1 200 mol d'aldéhyde par atome-g de rhodium et qu'on traite le matériau mis en oeuvre par de l'oxygène ou un gaz contenant de l'oxygène entre 60 et 120°C à pression atmosphérique ou sous pression accrue, en présence de 1,0 à 15 mol d'un acide monocarboxylique de 2 à 5 atomes de carbone par atome-g de rhodium et en présence d'un sel alcalin d'un acide monocarboxylique de 2 à 5 atomes de carbone et ensuite on extrait à l'eau pour séparer le rhodium sous forme de composé soluble dans l'eau et ensuite on sépare la phase aqueuse de la phase organique.

2. Procédé selon la revendication 1, caractérisé en ce que qu'on utilise de l'air comme gaz contenant de l'oxygène.

3. Procédé selon la revendication 1 ou 2, caractérisé en ce que, par kg de matériau mis en oeuvre, on utilise 1 à 10, notamment 2 à 6 mol d'oxygène.

4. Procédé selon l'une ou plusieurs des revendications 1 à 3, caractérisé en ce qu'on ajoute au matériau mis en oeuvre de l'acide acétique, de l'acide propionique, de l'acide n-butyrique, de l'acide i-butyrique ou de l'acide n-valérianique.

5. Procédé selon l'une ou plusieurs des revendications 1 à 4, caractérisé en ce qu'on ajoute au matériau mis en oeuvre par atome-g de rhodium 1,2 à 10, notamment 1,5 à 6 mol d'acide monocarboxylique.

6. Procédé selon l'une ou plusieurs des revendications 1 à 5, caractérisé en ce qu'on ajoute au matériau mis en oeuvre un sel de sodium ou de potassium de l'acide acétique, de l'acide propionique, de l'acide n-buryrique, de l'acide i-butyrique ou de l'acide n-valérianique.

7. Procédé selon l'une ou plusieurs des revendications 1 à 6, caractérisé en ce qu'on ajoute au matériau mis en oeuvre par atome-g de rhodium 10 à 100, notamment 12 à 50, de préférence 12 à 30 mol de sel alcalin d'acide monocarboxylique de 2 à 5 atomes de carbone.

8. Procédé selon l'une ou plusieurs des revendications 1 à 7, caractérisé en ce que le traitement du matériau mis en oeuvre par l'oxygène ou par un gaz contenant de l'oxygène a lieu entre 70 et 105, de préférence entre 80 et 100°C.

9. Procédé selon l'une ou plusieurs des revendications 1 à 8, caractérisé en ce que le traitement du matériau mis en oeuvre par l'oxygène ou par un gaz contenant de l'oxygène a lieu à des pressions de 0,2 à 1,0 MPa.

10. Procédé selon l'une ou plusieurs des revendications 1 à 9, caractérisé en ce que la concentration en rhodium du matériau mis en oeuvre est d'environ 300 ppm-masse et moins, notamment 20 à 200, de préférence 30 à 150 ppm-masse.